**Europäisches Patentamt**

**European Patent Office**

**Office européen des brevets**

(11) Veröffentlichungsnummer: **0 292 763**
**A2**

(12) **EUROPÄISCHE PATENTANMELDUNG**

(21) Anmeldenummer: 88107356.3

(22) Anmeldetag: 07.05.88

(51) Int. Cl.4: **C12N 15/00 , C12P 21/02 , C12N 9/22 , C12N 1/20 , //(C12N1/20,C12R1:19)**

(30) Priorität: **19.05.87 DE 3716722**

(43) Veröffentlichungstag der Anmeldung:
**30.11.88 Patentblatt 88/48**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Crause, Peter Dr.**
**Schopenhauerstrasse 31**
**D-6050 Offenbach(DE)**
Erfinder: **Hein, Friedrich, Dr.**
**Hugnagelstrasse 13**
**D-8000 München 21(DE)**
Erfinder: **Jansen, Hans Willi, Dr.**
**Drosselweg 1**
**D-6230 Frankfurt am Main 80(DE)**
Erfinder: **Uhlmann, Eugen, Dr.**
**Zum Talblick 31**
**D-6246 Glashütten/Taunus(DE)**

(54) **Gentechnologisches Verfahren zur Herstellung von Angiogeninen.**

(57) Fusionsproteine mit $\beta$-Galactosidase oder einem $\beta$-Galactosidase-Fragment mit mehr als 250 Aminosäuren, jedoch signifikant weniger als der gesamten Sequenz der $\beta$-Galactosidase, wobei störende Methionine und/oder Arginine und/oder Cysteine im $\beta$-Galactosidase-Anteil durch andere Aminosäuren ersetzt werden, und einem Angiogenin sind unlöslich und aus Bakterienzellen leicht zu isolieren. Angiogenin und seine Derivate können deshalb gewonnen werden, indem man das Strukturgen für das gewünschte Polypeptid im korrekten Leserahmen, nötigenfalls über einen Adaptor, an ein Gen für ein solches $\beta$-Galactosidase-Derivat über eine Regulationsregion koppelt, in einem Bakterium das unlösliche Fusionsprotein exprimiert, dieses nach einem Zellaufschluß isoliert und das gewünschte Angiogenin durch chemische oder enzymatische Spaltung freisetzt.

EP 0 292 763 A2

## Gentechnologisches Verfahren zur Herstellung von Angiogeninen

Die Beobachtung, daß Vaskularisierung eine Voraussetzung für das Wachstum der meisten soliden Tumore ist, führte zur Isolierung und Charakterisierung eines Angiogenin genannten Angiogenesefaktors aus dem Überstand von kultivierten HT29 Adenokarzinomzellen (Fett et al., Biochemistry 24 (1985) 5480-5486; Strydom et al., Biochemistry 24 (1985) 5486-5494). Angiogenin besteht aus einer nicht glykosylierten Polypeptidkette mit einer Molmasse von ~ 14200 D und einem isoelektrischen Punkt > 9,5. Ausgehend von der Proteinsequenzierung konnte mit spezifischen Sonden eine Angiogenin-cDNA und ein genomischer Angiogenin-Klon isoliert werden (Kurachi et al., Biochemistry 24 (1985) 5494-5499). Angiogenin weist eine 35 %ige Homologie zu Ribonuklease A (RNase A) auf, wobei 3 der 4 Disulfidbrücken der RNase A in exakt gleicher Position erhalten geblieben sind, ebenso wie die für die katalytische Aktivität der RNase A essentiellen Aminosäurereste His-12, Lys-41 und His-119. Weitere Arbeiten zeigten dann auch, daß Angiogenin RNase-Aktivität besitzt, die jedoch spezifischer ist als die von RNase A (Shapiro et al., Biochemistry 25 (1986) 3527-2532).

Die Möglichkeiten für einen therapeutischen Einsatz des Angiogenins liegen in der Beseitigung lokaler Durchblutungsstörungen, wobei eine Anwendung bei koronaren Durchblutungsstörungen und bei der Wundheilung, insbesondere von Knochen- und Bänderverletzungen und bei Hauttransplantationen, in Betracht kommt. Das zweite therapeutische Prinzip besteht in der Hemmung von Angiogenese, z.B. durch Antikörper gegen Angiogenin. Bei der Homologie des Angiogenins zu RNase A wäre auch an synthetische niedermolekulare Inhibitoren zu denken. Dieses Prinzip könnte bei der Diagnose und Therapie solider Tumore und ihrer Metastasen (Synergismus mit Chemotherapie), bei rheumatischen Arthritiden, diabetischen Spätschäden (Retinopathie) und anderen Krankheitsbildern Anwendung finden.

Um alle diese therapeutischen Möglichkeiten auszuschöpfen, ist es jedoch zunächst notwendig, biologisch aktives Angiogenin auf vorteilhafte Weise in den Mengen zu isolieren, die weitergehende Studien überhaupt erst ermöglichen.

Bei der gentechnologischen Herstellung von Polypeptiden in Bakterien wird häufig das Strukturgen für das gewünschte Polypeptid im Leserahmen an das Gen für das bakterieneigene Polypeptid $\beta$-Galactosidase gekoppelt. Das Bakterium produziert dann ein Fusionsprotein, bei dem an den Carboxy-Terminus der $\beta$-Galactosidase das gewünschte Polypeptid über den Amino-Terminus gebunden ist. Es ist auch bekannt, daß man bei diesem Verfahren nicht das Gen für die gesamte $\beta$-Galactosidase ausnützt (EP-A 0 001 930 und 0 012 494). Hierbei wurde jedoch nur ein extrem kurzes Stück oder aber im wesentlichen die gesamte Sequenz der $\beta$-Galactosidase eingesetzt.

Es wurde bereits ein Verfahren zur Herstellung eines genetisch codierbaren Polypeptids vorgeschlagen (nicht vorveröffentlichte deutsche Patentanmeldung P 38 05 150.8), wobei man das Strukturgen für dieses Polypeptid im korrekten Leserahmen über das Gen für die $\beta$-Galactosidase oder ein Fragment der $\beta$-Galactosidase an eine Regulationsregion koppelt, diese Genstruktur in ein Bakterium einbringt, darin ein unlösliches Fusionsprotein exprimiert, dieses nach einem Zellaufschluß isoliert und das gewünschte Polypeptid durch chemische oder enzymatische Spaltung gewinnt, das dadurch gekennzeichnet ist, daß im Gen für die $\beta$-Galactosidase bzw. für das Fragment der $\beta$-Galactosidase Codons für Methionin und/oder Arginin und/oder Cystein ganz oder teilweise durch Codons anderer Aminosäuren ersetzt sind.

Es wurde nun gefunden, daß nach diesem Verfahren Angiogenin und seine Derivate besonders vorteilhaft zugänglich sind. Vorteilhafte Ausgestaltungen der Erfindung werden im folgenden näher erläutert.

Die Figuren veranschaulichen die Erfindung wie folgt:

Fig. 1 zeigt Konstruktionen von verkürzten $\beta$-Galactosidase-Sequenzen ohne (A 1. - 5.) und mit (B 1. - 5.) Linker.

Fig. 2 zeigt die Konstruktion des Plasmids pWZ RI (ein pBR 322-Derivat mit $\beta$-Galactosidase-Fragmenten aus pUC 9 und pUR 270).

Fig. 3 zeigt die Konstruktion des Plasmids pLZΔ.

Fig. 4 zeigt die Konstruktion des Plasmids pLZP RI dMdC aus pWZIP dMdC.

Fig. 5 zeigt die Konstruktion der Plasmide pLZPWB1 dMdC und pLZPWB2 dMdC.

Fig. 6 zeigt schematisch die Ligationsreaktion, die zu einem synthetischen Angiogenin führt, wobei R Reinigung, L Ligation und das Kreis-symbol die 5'-Phosphatgruppe bedeuten.

Die Figuren sind nicht maßstabsgerecht.

Zunächst werden Einzelheiten des Verfahrens gemäß der deutschen Patentanmeldung P 38 05 150.8 zur Herstellung der Fusionsproteine geschildert:

Der $\beta$-Galactosidase-Anteil in den Fusionsproteinen weist vorteilhaft über 250 Aminosäuren, jedoch signifikant weniger als die gesamte $\beta$-Galactosidasesequenz auf. Bewährt haben sich $\beta$-Galactosidasefrag-

mente von etwa 300 bis etwa 800, vorzugsweise etwa 320 bis etwa 650 Aminosäuren, die zweckmäßig einen aminoterminalen und/oder carboxyterminalen Anteil der $\beta$-Galactosidase enthalten. Dieses $\beta$-Galacto- sidasegen-Fragment ist im korrekten Leserahmen an eine Regulationsregion und, nötigenfalls über einen Adaptor, an das Strukturgen für Angiogenin gebunden.

Der Adaptor, der gegebenenfalls auch entfallen kann, kodiert hierbei vorteilhaft für eine oder mehrere Aminosäuren, die vor dem Amino-Terminus des Angiogenin liegen und dessen leichte - chemische oder enzymatische - Abtrennung vom $\beta$-Galactosidase-Fragment erlauben. Wenn das gewünschte Angiogenin- Derivat beispielsweise kein Methionin enthält bzw. gentechnologisch so variiert wurde, daß es kein Methionin enthält, so wählt man vorteilhaft als Aminosäure vor dem Amino-Terminus des gewünschten Polypeptids Methionin, worauf durch Chlorcyan- oder Bromcyan-Spaltung das gewünschte Polypeptid vom $\beta$-Galactosidase-Anteil abgetrennt werden kann.

Das erfindungsgemäß eingesetzte Verfahren hat den Vorteil, daß das gebildete Fusionsprotein unlöslich ist und somit nach einem Zellaufschluß leicht isolierbar ist. Bei den bevorzugten Ausführungsformen kann andererseits das Fusionsprotein aufgrund des relativ geringen $\beta$-Galactosidase-Anteils in größeren Mengen im Bakterium akkumulieren. Außerdem wird das Fusionsprotein wegen des Anteils an einem bakterieneigenen Protein vom Bakterium nicht nennenswert abgebaut und erlaubt somit entsprechend längere Induktionsperioden und dadurch eine höhere Ausbeute.

Erfindungsgemäß kann somit ein Angiogenin dadurch gewonnen werden, daß man das Strukturgen für dieses Polypeptid im korrekten Leserahmen, nötigenfalls über einen Adaptor, mit dem Gen für $\beta$- Galactosidase oder ein $\beta$-Galactosidase-Fragment von vorzugsweise über 250 Aminosäuren, jedoch signifikant weniger als die gesamte $\beta$-Galactosidase-Sequenz, an eine Regulationsregion koppelt, diese Genstruktur in ein Bakterium einbringt, darin ein unlösliches Fusionsprotein exprimiert, dieses nach einem Zellaufschluß isoliert und das gewünschte Polypeptid durch chemische oder enzymatische Spaltung gewinnt.

Die Regulationsregion kann natürlich, insbesondere bakterieneigen, chemisch-synthetisch oder eine Hybridregion sein, beispielsweise der Fusionspromotor tac. Die Regulationsregionen enthalten zusätzlich einen Operator, z.B. den lac-Operator, und 6 bis 14 Nucleotide vor dem Methionin-Codon der $\beta$- Galactosidase-Fragmente eine ribosomale Bindungsstelle.

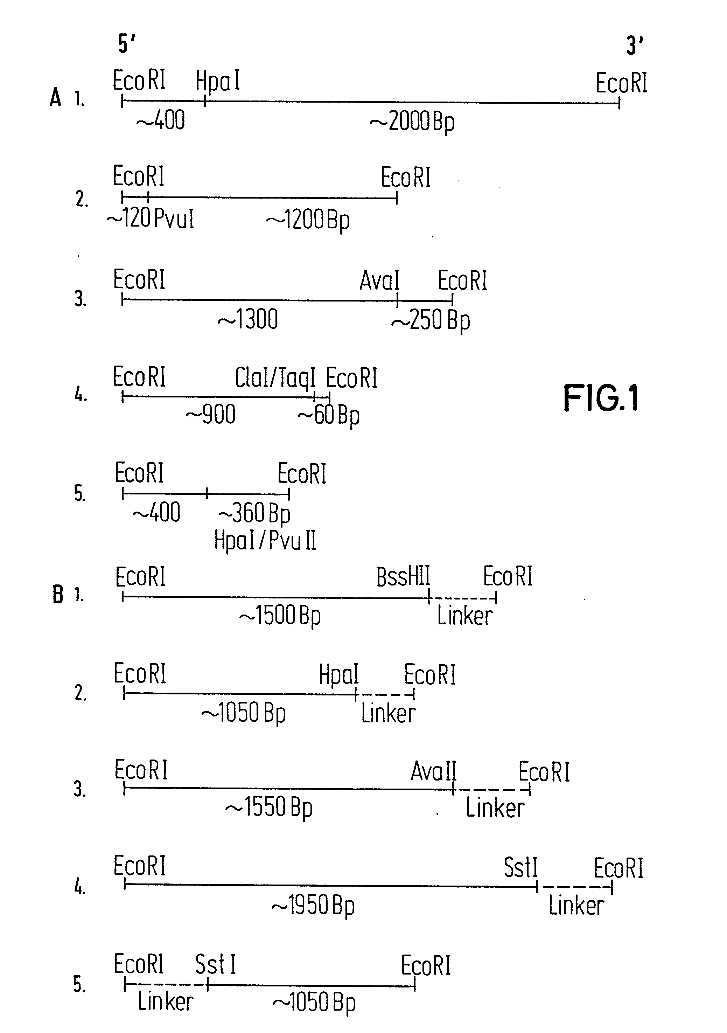

Das $\beta$-Galactosidase-Fragment besteht vorteilhaft aus einer Fusion einer aminoterminalen und carboxyterminalen Teilsequenz. Hierdurch wird der Anteil der $\beta$-Galactosidase im Fusionsprotein erheblich reduziert. Außerdem erlaubt diese Konstruktion den Austausch verschiedener Regulationssysteme ohne besonderen Aufwand. Es können jedoch auch ausschließlich aminoterminale oder überwiegend carboxyterminale Sequenzen der $\beta$-Galactosidase eingesetzt werden. Für diese Konstruktionen von $\beta$-Galactosidaseverkür- zungen kann von natürlichen Restriktionsenzym-Schnittstellen Gebrauch gemacht werden. Es können aber auch Konstruktionen mit chemisch-synthetischen Linkern bzw. Adaptoren verwendet werden, die einen korrekten Leserahmen - frei von Stop-Codons - gewährleisten und gegebenenfalls ein ATG-Startcodon enthalten. Die Figur 1 A zeigt $\beta$-Galactosidasegen-Fragmente, bei denen von natürlichen Restriktionsenzymschnittstellen Gebrauch gemacht wurde, die Figur 1 B solche unter Verwendung eines Linkers.

Weitere Modifikationen des $\beta$-Galactosidase-Fragmentes können sich von Fall zu Fall einzeln oder in Kombination als vorteilhaft erweisen.

Bei durch Chlorcyan- oder Bromcyan-Spaltung vom $\beta$-Galactosidase-Fragment abzutrennenden Poly- peptiden erleichtert es die Reinigung der letzteren, wenn man durch gezielte in-vitro-Mutagenese einige oder vorteilhaft alle Codons der störenden Methionin-Reste durch Codons für andere Aminosäuren, vorzugsweise Leucin oder Isoleucin, ersetzt.

Die Spaltung führt bei einem so modifizierten Fusionsprotein neben dem gewünschten Polypeptid zu einer verringerten Zahl an $\beta$-Galactosidase-Spaltfragmenten, die so gewählt werden können, daß sie sich aufgrund ihrer Größe und/oder Ladung leicht vom gewünschten Polypeptid trennen lassen.

Bei Polypeptiden, die durch saure Spaltung der Peptidbindung zwischen Asparaginsäure und Prolin vom $\beta$-Galactosidase-Fragment abgetrennt werden sollen, kann es von Vorteil sein, die entsprechenden Codons im $\beta$-Galactosidasegen-Fragment durch gezielte in-vitro-Mutagenese so zu verändern, daß an diesen Stellen keine saure Spaltung mehr möglich ist, vorzugsweise durch Umwandlung des Codons für Asparaginsäure in ein Codon für Glutaminsäure.

Bei Polypeptiden, für deren Aktivität die Ausbildung von Disulfidbrücken von Bedeutung ist, erweist es sich als generell nützlich, die Codons für Cystein, die im $\beta$-Galactosidase-Fragment vorhanden sind, durch gezielte in-vitro-Mutagenese in Codons für andere Aminosäuren, vorzugsweise Serin, umzuwandeln, um so eine mögliche Ausbildung falscher Disulfidbrücken zwischen dem $\beta$-Galactosidase-Fragment und dem Polypeptid unmöglich zu machen.

Der Adaptor zwischen dem $\beta$-Galactosidase-Fragment und dem Strukturgen für das gewünschte Polypeptid, der in günstigen Fällen entfallen kann, kodiert - unmittelbar vor dem Amino-Terminus des gewünschten Polypeptids - für eine Aminosäure oder eine Folge von Aminosäuren, die eine leichte Abtrennung des gewünschten Polypeptids vom β-Galactosidaseanteil erlauben. Wie bereits erwähnt, kann diese Aminosäure Methionin sein, was eine einfache Bromcyan-Spaltung erlaubt, sofern das gewünschte Polypeptid kein Methionin enthält bzw. die entsprechenden Codons gentechnologisch verändert wurden. Ein Beispiel für einen solchen Adaptor weist die folgende Nucleotidsequenz auf:

```
5'        AAT TAT GAA TTC GCA ATG
(Eco RI)      TA CTT AAG CGT TAC
```

Eine zusätzliche Erleichterung der verschiedenen Abspaltungsmodalitäten - chemisch oder enzymatisch - kann durch die sterische Separierung des gewünschten Polypeptids vom β-Galactosidase-Anteil erreicht werden. Hierzu werden über einen speziellen, chemisch synthetisierten Adaptor die Codons für eine Poly-Aminosäure zwischen den β-Galactosidase-Anteil und das Polypeptid eingefügt. Als Aminosäuren können unter dem Aspekt der unterschiedlichen Strukturierung dieses Poly-Aminosäure-"Arms" generell alle genetisch codierbaren Aminosäuren eingesetzt werden, beispielsweise kleine ungeladene Aminosäuren wie Glycin, Alanin, Serin oder Prolin oder geladene wie Asparaginsäure und Glutaminsäure einerseits oder Lysin und Arginin andererseits. Die Poly-Aminosäurekette umfaßt zweckmäßig 5 bis 30, vorzugsweise 10 bis 24, insbesondere 15 bis 20 Aminosäuren. Je nach Wahl der Poly-Aminosäuren läßt sich eine direkte Rückfaltung des gewünschten Genproduktes im Verbund mit dem β-Galactosidase-Anteil erreichen.

Das Strukturgen für das gewünschte Angiogenin kann in an sich bekannter Weise chemisch synthetisiert werden. Vorteilhaft sind chemisch synthetisierte Gene, die auf den speziellen Codongebrauch der Wirtszelle abgestimmt sind, wie es beispielsweise in den deutschen Offenlegungsschriften 33 27 007 (Derivate des Wachstumshormon-Releasing Factor), 33 28 793 (Derivat des Sekretin), 34 09 966 (Human-γ-Interferon), 34 14 831 (Derivate des Human-γ-Interferon), 34 19 995 (Interleukin-2 und Derivate) und 34 29 430 (Hirudin-Derivate) beschrieben oder in der deutschen Patentanmeldung P 36 32 037.4 (Calcitonin) vorgeschlagen wurde.

Zu diesem Zweck wird auf chemisch-synthetischem Weg ein Gen für ein Angiogenin hergestellt, das mehrere Vorteile aufweist. Bei dem synthetischen Gen (Tab. 1) sind die Präferenzen von E. coli berücksichtigt. Innerhalb des Strukturgens ist eine Reihe von singulären Erkennungssequenzen für Restriktionsendonukleasen eingebaut, die Zugang zu Teilsequenzen des Angiogenins schaffen sowie die Veränderung des Gens durch Mutationen erleichtern. Außerdem ist das Codon für das einzige im natürlichen Angiogenin enthaltene Methionin in ein Leucin-, Isoleucin- oder Valin-Codon umgewandelt, was ohne Verlust an biologischer Aktivität das vorteilhafte Abtrennen des Angiogenins vom β-Galactosidase-Fragment des Fusionsproteins durch Bromcyanspaltung erlaubt.

Der Einbau der Genstruktur, bestehend aus Regulationsregion, β-Galactosidase-Fragment, gegebenenfalls Adaptor und Strukturgen, in einen geeigneten Vektor, das Einbringen des so gewonnenen Hybridvektors in eine geeignete Wirtszelle, die Kultivierung der Wirtszellen, der Zellaufschluß, die Isolierung und Spaltung des Fusionsproteins sowie die Isolierung des gewünschten Polypeptids sind allgemein bekannt. Es kann hierzu auf die verbreiteten Lehr- und Handbücher verwiesen werden.

Bevorzugte Vektoren sind Plasmide, insbesondere die mit E. coli verträglichen Plasmide wie pBR 322, pBR 325, pUC 8 und pUC 9 sowie andere handelsübliche bzw. allgemein zugängliche Plasmide. Der bevorzugte bakterielle Wirt ist E. coli.

In den folgenden Beispielen wird die Erfindung näher erläutert.

**Beispiel 1**

20 μg des handelsüblichen Plasmids pUC 9 (vgl. Vieira et al., Gene 19 (1982) 259 - 268; The Molecular Biology Catalogue, Pharmacia P-L Biochemicals, 1984, Appendix, S. 40) werden einer Doppelverdauung mit den Restriktions-Endonukleasen Eco RI und Pvu I unterzogen und ein DNA-Fragment von 123 Basenpaaren (Bp) Länge gelelektrophoretisch abgetrennt. Dieses Fragment umfaßt einen Teil der aminoterminalen Codierungssequenz der β-Galactosidase.

Zur Isolierung des carboxyterminalen Anteils des β-Galactosidasegens bis zur natürlichen Eco RI-Schnittstelle werden 20 μg des Plasmids pUR 270 (Rüther und Müller-Hill, EMBO J. 2 (1983) 1791-1794) zunächst mit Eco RI verdaut und anschließend mit dem Enzym Pvu I einer Partialverdauung unterworfen. Durch Elektrophorese auf einem 5 %igen Polyacrylamidgel wird ein DNA-Fragment von 2895 Bp abgetrennt und isoliert.

Die aminoterminalen und carboxyterminalen DNA-Fragmente des β-Galactosidasegens werden im Lauf von 6 Stunden bei 16°C zusammenligiert und das Ligierungsprodukt mit Ethanol gefällt. Die gefällte und resuspendierte DNA wird mit Eco RI geschnitten und noch einmal auf einem 5 %igen Polyacrylamidgel fraktioniert. Das DNA-Fragment der Länge 3018 Bp wird durch Elektroelution aus dem Gel isoliert und in die Eco RI-Schnittstelle des Plasmids pBR 322 ligiert. Das so erhaltene Hybridplasmid wird als pWZ RI bezeichnet.

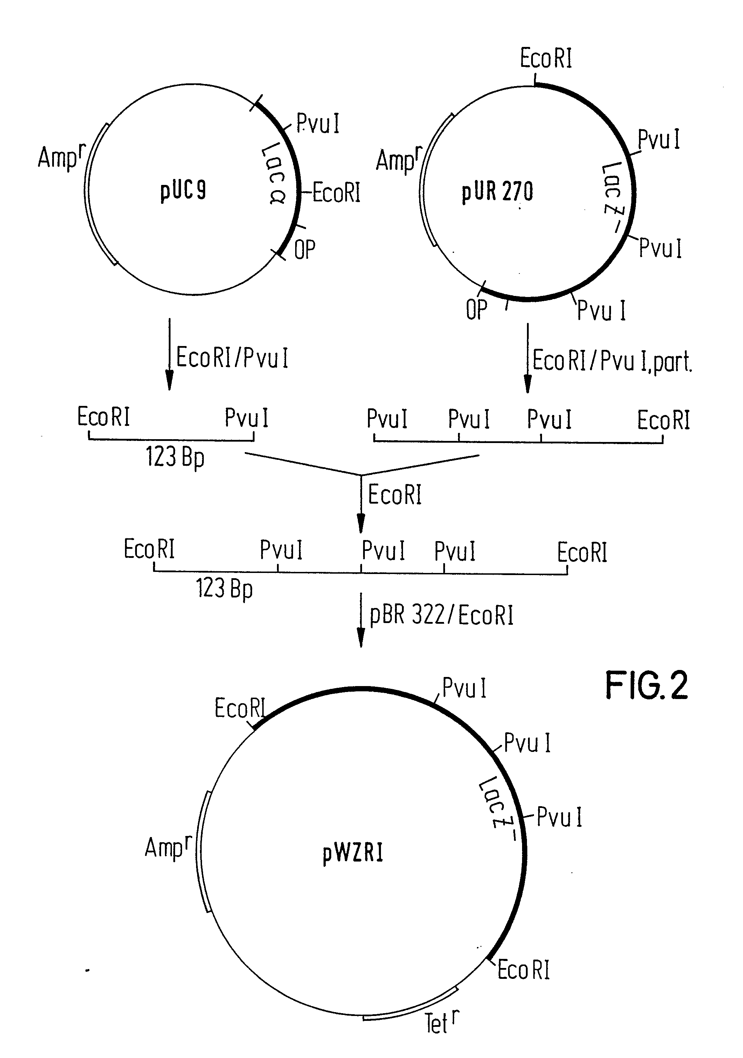

Die vorstehend beschriebenen Reaktionsschritte sind in der Figur 2 dargestellt. Die Einzelmaßnahmen wurden in bekannter Weise (Maniatis et al., Molecular Cloning, Cold Spring Harbor 1982) durchgeführt.

Das Plasmid pWZ RI wird in E. coli transformiert, dort amplifiziert und reisoliert. Durch Verdauung mit Eco RI kann das amino- und carboxyterminal verkürzte β-Galactosidase-Genfragment herausgespalten und präparativ isoliert werden. Über die bekannten Restriktionsenzym-Schnittstellen können weitere Verkürzungen konstruiert und in geeignete Expressionsplasmide eingefügt werden. Die Figur 1 A zeigt solche Verkürzungen.

Die Figur 1 B zeigt Konstruktionen mit einem chemisch-synthetischen Linker.

Sowohl die Konstruktionen aus Figur 1 A als auch die aus Figur 1 B sind so gewählt, daß der Leserahmen der verkürzten β-Galactosidase kontinuierlich übergeht in den Leserahmen des gewünschten carboxyterminalen Polypeptides. Die chemisch-synthetischen Linker der Figur 1 B können beliebig gestaltet sein, müssen jedoch selbstverständlich den Stopcodon-freien Leserahmen und gegebenenfalls ein ATG-Startcodon gewährleisten und die gewünschten Restriktionsenzym-Schnittstellen aufweisen.


## Beispiel 2

Eine vorteilhafte Verkürzung und Modifikation des durch Eco RI-Spaltung aus pWZ RI erhältlichen Fragmentes des β-Galactosidase Gens ist wie folgt zugänglich:

10 μg des Plasmids pWZ RI (Fig. 2) werden mit den Restriktionsenzymen Eco RI und Pvu I geschnitten und auf einem 7,5%igen Polyacrylamidgel aufgetrennt. DNA-Fragmente von 123 und 1222 Bp Länge werden isoliert. Äquimolare Mengen der beiden DNA-Fragmente werden anschließend für 6 Stunden bei 10°C zusammenligiert und mit Eco RI nachverdaut. Das so erhaltene Ligationsgemisch wird auf einem 7,5%igen Polyacrylamidgel aufgetrennt, und die DNA-Bande mit einer Länge von 1345 Bp wird präparativ isoliert. Dieses DNA-Fragment wird in den mit Eco RI geöffneten und anschließend dephosphorylierten Vektor pBR 322 einligiert. Das so erhaltene Plasmid wird pWZP RI genannt.

Zur Entfernung der 8 in pWZP RI enthaltenen Codons für Methionin (M1-M8) und der 6 in diesem Plasmid vorhandenen Codons für Cystein (C1-C6) wird 1 μg DNA von pWZP RI mit Eco RI gespalten. Das 1345 Bp große Fragment wird in den mit Eco RI geöffneten und anschließend dephosphorylierten Phagenvektor M13mp19am (Patschinsky et al., J. Virol. 59 (1986) 341-353) einligiert. Der nach Transfektion von E. coli JM101 erhaltene Phage wird als MWZPam bezeichnet. Als erster Schritt zur Entfernung der Codons für Methionin wird eine gezielte in-vitro-Mutagenese nach der "gapped duplex"-Methode (Kramer et al., Nucl. Acids Res. 12 (1984) 9441-9456) durchgeführt, wobei aufgrund der guten Effizienz dieser Methode (im Mittel 70%) die 4 Oligonucleotide dM5 -dM8 (Tab. 2) als mutagene "Primer" Verwendung finden. Die ssDNA von 12 der resultierenden Phagen wird sequenziert, wobei neben dem normalen 17mer-"Primer" auch dM7 und dC5 (Tab. 2) als "Primer" für die Sequenzierung verwendet werden. 2 der 12 DNAs weisen alle 4 gewünschten Mutationen auf, d.h. die Codons für M5-M8 sind in Codons für Isoleucin verändert worden. Diese Phagen werden als MWZP dM5,8 bezeichnet. Für die Entfernung der restlichen Methionin-Codons wird die RF-DNA von MWZP dM5,8 mit Eco RI gespalten und das 1345 Bp große DNA-Fragment in dephosphorylierten M13mp19am einkloniert. Der so erhaltene Phage wird als MWZP dM5,8am bezeichnet. Die ssDNA dieses Phagen wird einer weiteren in-vitro-Mutagenese mit dM1-dM4 als mutagenen "Primern" unterworfen. Die ssDNA von 12 der resultierenden Phagen wird sequenziert, und 3 der 12 Phagen weisen alle gewünschten Mutationen auf, d. h. die Codons für M1-M3 sind in Codons für Leucin und das Codon für M4 in ein Codon für Isoleucin verändert worden. Diese Phagen werden als MWZPdM bezeichnet. Durch Einklonieren des 1345 Bp Eco RI-Fragments aus der RF-Form dieser Phagen in den dephosphorylierten Vektor pBR 322 wird das Plasmid pWZP dM erhalten. Zur zusätzlichen Umwandlung der Codons für Cystein in Codons für Serin wird das 1345 Bp Eco RI-Fragment aus pWZP dM isoliert und in den dephosphorylierten Phagenvektor M13mp19am einkloniert. Die ssDNA des so erhaltenen Phagen MWZP dMam wird einer in-vitro-Mutagenese mit dC1-dC6 als mutagenen "Primern" unterzogen. Die ssDNA von 24 der erhaltenen Phagen wird sequenziert, wobei sich 4 der Phagenklone, die als MWZP dMdC bezeichnet werden, als die richtigen erweisen, bei denen alle Codons für Cystein in Codons für Serin umgewandelt wurden. Die RF-DNA dieser Phagen wird mit Eco RI gespalten und das 1345 Bp Eco RI-Fragment wird in den dephosphorylierten Vektor pBR 322 einkloniert, wobei man das Plasmid pWZP dMdC erhält.

**Beispiel 3**

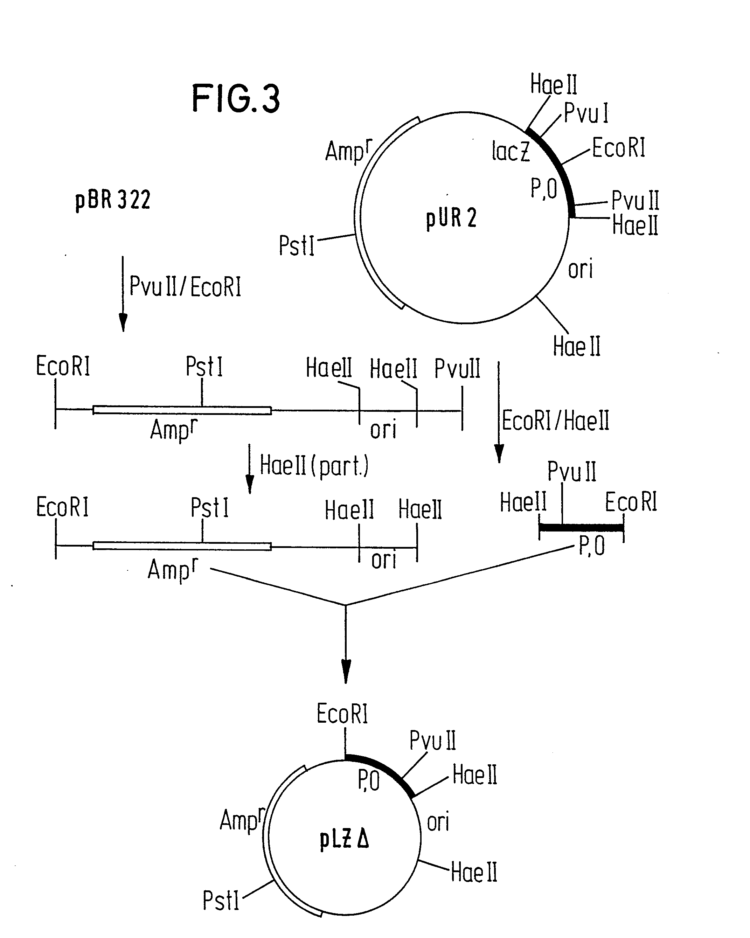

Das Plasmid pLZΔ, in das sich die verkürzten und/oder modifizierten Versionen des β-Galactosidasegens vorteilhaft einklonieren lassen, ist wie folgt zugänglich (Fig. 3):

10 μg des Plasmids pBR 322 werden mit den Restriktionsendonukleasen Eco RI und Pvu II geschnitten und auf einem 1 %igen Agarosegel aufgetrennt. Das 2293 Bp große DNA-Fragment wird gereinigt, mit Hae II partiell gespalten und erneut auf einem 1 %igen Agarosegel elektrophoretisiert. Das 2017 Bp große DNA-Fragment wird isoliert und mit dem nach Spaltung mit Eco RI und Hae II aus pUR 2 (Rüther, Mol. Gen. Genet. 178 (1980) 475-477) erhaltenen 225 Bp großen DNA-Fragment, das den Promotor/Operator des lac-Operons, eine ribosomale Bindungsstelle und die ersten 5 Codons des lac Z-Gens (incl. Start-Codon) enthält, bei 12°C über Nacht ligiert. Nach Transformation kompetenter E. coli-Zellen erhält man das Plasmid pLZΔ.

**Beispiel 4**

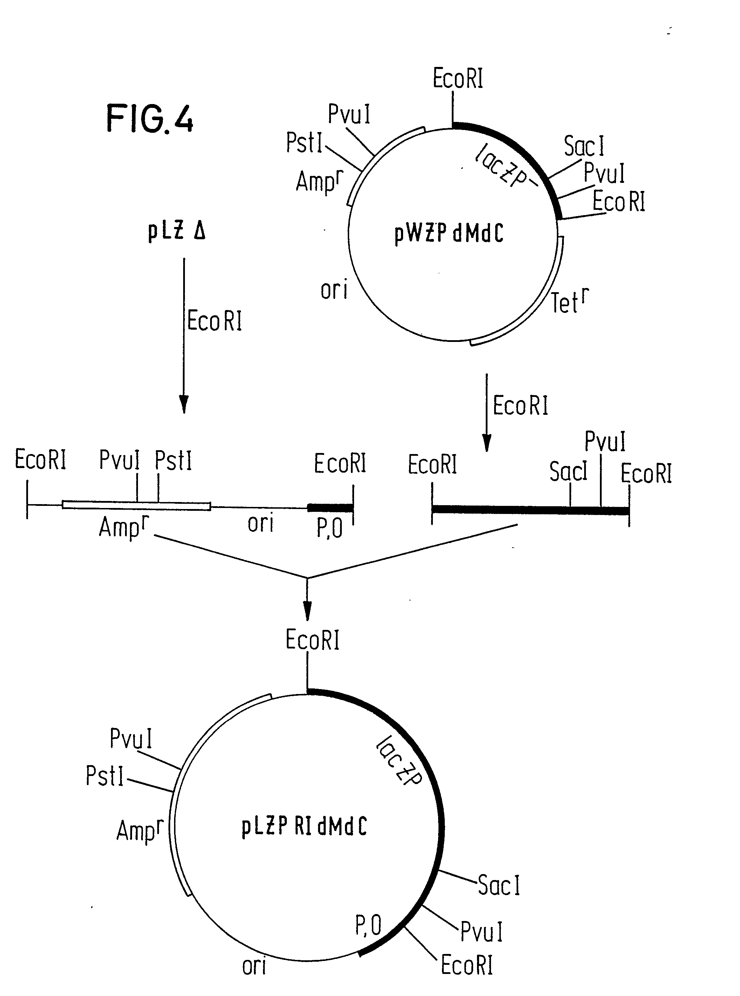

Plasmide, die sich aufgrund von multiplen Klonierungsstellen für die Expression von Polypeptiden als Fusionsproteine mit β-Galactosidase-Verkürzungen eignen, erhält man wie folgt (Fig. 4):

1 μg des Plasmids pLZΔ (Fig. 3) wird mit Eco RI geöffnet, dephosphoryliert und bei 12°C über Nacht mit dem modifizierten 1345 Bp großen Eco RI-Fragment aus pWZP dMdC (Beispiel 2) ligiert. Nach Transformation kompetenter E. coli-Zellen und Selektion auf Expression der β-Galactosidase und korrekte Verteilung der Schnittstellen verschiedener Restriktionsendonukleasen erhält man das Plasmid pLZP RI dMdC (Fig. 4).

Für das Einklonieren einer multiplen Klonierungsstelle in die Eco RI-Stelle am 3'-Ende des β-Galactosidase-Gens erweist es sich als vorteilhaft, die Eco RI-Stelle am 5'-Ende durch gezielte in vitro-Mutagenese zu entfernen. Dazu wird das 1767 Bp große Pst I-Sac I-Fragment in M13mp19am einkloniert und mit dem mutagenen Primer

5'-GCCAGTGAATCCGTAATCATGG-3'

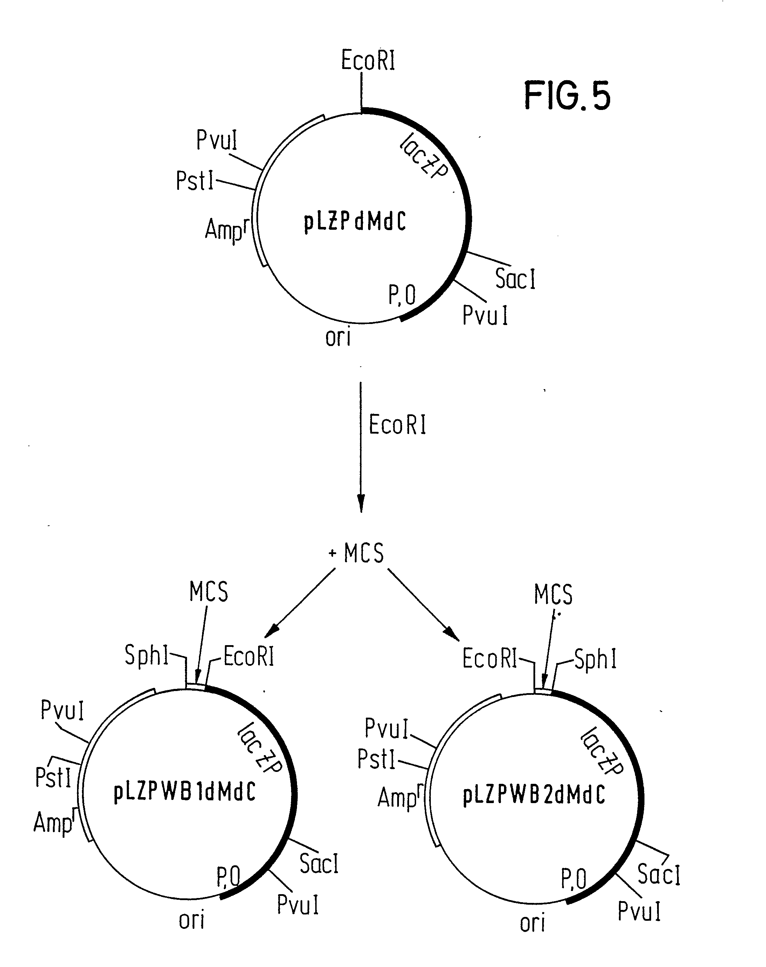

einer in vitro-Mutagenese nach der "gapped duplex"-Methode unterzogen. Die DNA von 2 von 4 untersuchten Phagenklonen weist nach Sequenzieren und Restriktionsanalyse das gewünschte Fehlen der Schnittstelle für Eco RI auf. Aus der RF-DNA dieser Phagenklone wird das 1767 Bp große Pst I-Sac I-Fragment isoliert und bei 16°C für 4 Stunden mit dem 1820 Bp großen Pst I-Sac I-Fragment aus pLZP RI dMdC ligiert. Nach Transformation kompetenter E. coli-Zellen erhält man das Plasmid pLZP dMdC (Fig. 5).

· Für den Einbau der multiplen Klonierungsstelle werden 2 μg von pLZP dMdC mit Eco RI geöffnet, dephosphoryliert und mit folgender chemisch synthetisierter DNA-Sequenz (MCS) ligiert, die eine Vielzahl von Restriktionsenzym-Schnittstellen enthält:

|  | (Eco RI) | | Sac I | | Sma I | | Bam HI | | Xba I | | Sal I | |
|---|---|---|---|---|---|---|---|---|---|---|---|---|
| 5' | AA | TTC | GAG | CTC | GCC | CGG | GGA | TCC | TCT | AGA | GTC | GAC |
| 3' |  | G | CTC | GAG | CGG | GCC | CCT | AGG | AGA | TCT | CAG | CTG |

|  Pst I | | | | Hind III | | Nru I | | Ava III | | Bgl II | |
|---|---|---|---|---|---|---|---|---|---|---|---|
| CTG | CAG | CCC | AAG | CTT | CGC | GAT | GCA | TCA | GAT | CTA |
| GAC | GTC | GGG | TTC | GAA | GCG | CTA | CGT | AGT | CTA | GAT |

| Nco I | | Sph I | | (Eco RI⁻) | |
|---|---|---|---|---|---|
| CCA | TGG | CAT | GCC |  | 3' |
| GGT | ACC | GTA | CGG | TTA A | 5' |

Auf diese Weise erhält man nach Transformation kompetenter E. coli-Zellen die Plasmide pLZPWB1 dMdC und pLZPWB2 dMdC (Fig. 5), die sich durch die Orientierung ihrer MCS-Sequenz voneinander unterscheiden.

**Beispiel 5**

Das Einklonieren eines synthetischen Angiogenin-Gens (Tab. 1), das sich vom normalen menschlichen Angiogenin-Gen vor allem durch den Ersatz des Codons für Met-30 gegen ein Codon für Leucin, Isoleucin oder Valin unterscheidet, in den oben beschriebenen Vektor pLZPWB2 dMdC wird wie folgt durchgeführt:

Das synthetische Gen für Angiogenin wird aus 12 Oligonucleotid-Bausteinen (s. Tab. 3) zusammengesetzt.

Am Beispiel des Genbausteins Ia, der die Nucleotide 1 - 49 des codierenden Strangs umfaßt, wird die Synthese der Genbausteine erläutert. Für die Festphasensynthese wird das am 3'-Ende stehende Nucleosid, im vorliegenden Falle also Adenosin (Nucleotid Nr. 49), über die 3'-Hydroxyfunktion kovalent an einen Träger gebunden verwendet. Trägermaterial ist mit langkettigen Aminoalkylresten funktionalisiertes CPG ("Controlled Pore Glass").

In den folgenden Syntheseschritten wird die Basenkomponente als 5'-O-Dimethoxytritylnucleosid-3'-phosphorigsäure-$\beta$-cyanoethylester-dialkylamid eingesetzt, wobei das Adenin als $N^6$-Benzoyl-Verbindung, das Cytosin als $N^4$-Benzoyl-Verbindung, das Guanin als $N^2$-Isobutyryl-Verbindung und das Thymin ohne Schutzgruppe vorliegen.

25 mg des polymeren Trägers, der 0.2 $\mu$mol $N^6$-Benzoyladenosin gebunden enthält, werden nacheinander mit folgenden Agentien behandelt:

A) Acetonitril

B) 3 % Trichloressigsäure in Dichlormethan

C) Acetonitril

D) 5 $\mu$mol des entsprechenden Nucleosid-3'-O-phosphits und 25 $\mu$mol Tetrazol in 0,15 ml wasserfreiem Acetonitril

E) Acetonitril

F) 20 % Acetanhydrid in Tetrahydrofuran mit 40 % Lutidin und 10 % Dimethylaminopyridin

G) Acetonitril

H) 3 % Jod in Lutidin/Wasser/Tetrahydrofuran im Volumenverhältnis 10 : 1 : 40

Unter "Phosphit" wird hierbei der 2'-Desoxyribose-3'-monophosphorigsäure-mono-$\beta$-cyanoethylester verstanden, wobei die dritte Valenz durch einen Diisopropylaminorest abgesättigt ist. Die Ausbeuten der einzelnen Syntheseschritte können jeweils nach der Detritylierungsreaktion B) spektrophotometrisch durch Messung der Absorption des Dimethoxytritylkations bei der Wellenlänge von 496 nm bestimmt werden.

Nach abgeschlossener Synthese erfolgt die Abspaltung der Dimethoxytritylgruppe wie in A) bis C) beschrieben. Durch die Behandlung mit Ammoniak wird das Oligonucleotid vom Träger gespalten und zugleich werden die $\beta$-Cyanoethylgruppen eliminiert. Eine 16-stündige Behandlung der Oligomeren mit konzentriertem Ammoniak bei 50° C spaltet die Aminoschutzgruppen der Basen quantitativ ab. Das so erhaltene Rohprodukt wird durch Polyacrylamid-Gelelektrophorese gereinigt.

Zur Phosphorylierung der Oligonucleotide am 5′-Terminus werden je 1 nmol der Oligonucleotide 1b bis 6a mit 5 nmol Adenosintriphosphat mit vier Einheiten T4 Polynucleotid-Kinase in 20 μl 50 mM Tris-HCl-Puffer (pH 7,6), 10 mM Magnesiumchlorid und 10 mM Dithiotreitol (DTT) 30 Minuten bei 37° C behandelt. Das Enzym wird durch fünfminütiges Erhitzen auf 95° C desaktiviert. Die Oligonucleotide 1a und 6b, welche in der DNA-Sequenz I die "überhängenden" einzelsträngigen Sequenzen bilden, werden nicht phosphoryliert. Dies verhindert bei der nachfolgenden Ligation die Ausbildung größerer Genfragmente als sie der DNA-Sequenz I entsprechen.

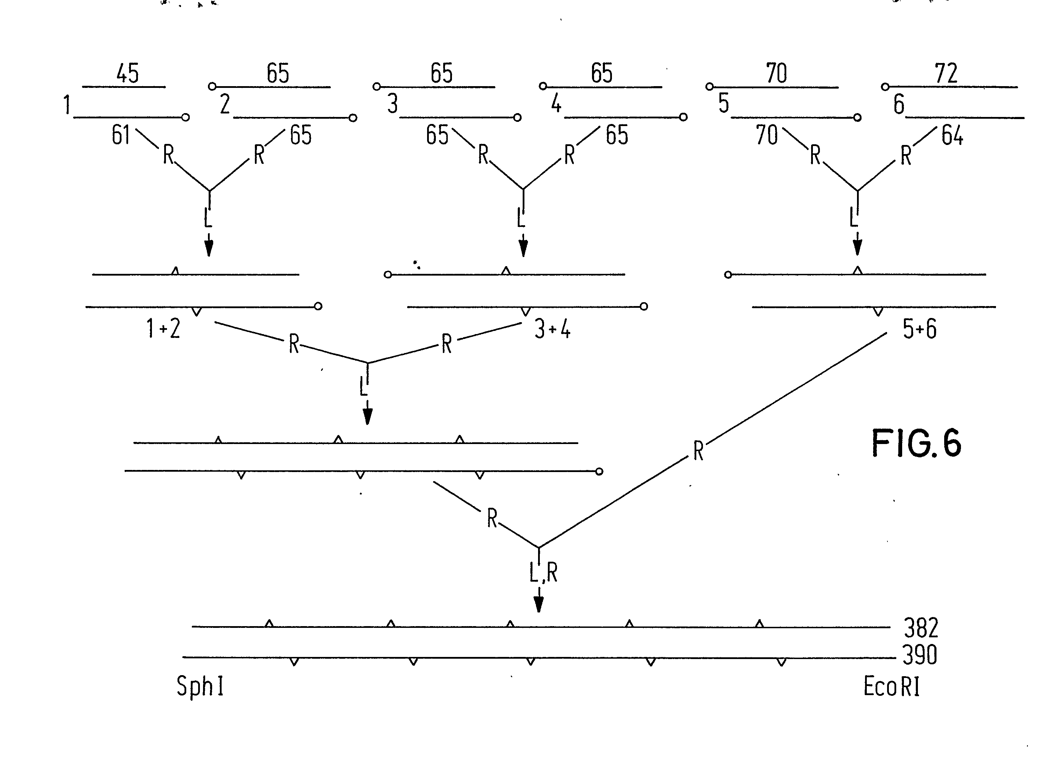

Die Oligonucleotide 1a bis 6b (vgl. Tab. 3) werden wie folgt zum Gesamtgen ligiert (Fig. 6): Je 1 nmol der Oligonucleotide 1a und 1b bis 6a und 6b werden paarweise hybridisiert, indem diese in jeweils 20 μl 50 mM Tris-HCl-Puffer (pH 7,6), 10 mM Magnesiumchlorid und 10 mM DTT gelöst werden, diese Lösung 5 Minuten auf 95° C erhitzt und binnen 2 Stunden auf Raumtemperatur abgekühlt wird. Dabei werden die Oligonucleotide 1b bis 6a in Form ihrer 5′-Phosphate eingesetzt. Zur weiteren Verknüpfung der gebildeten bihelikalen DNA-Fragmente werden die Lösungen derselben wie in Fig. 6 gezeigt durch Ligation zum vollständigen Gen zusammengesetzt.

Die Reinigung des Gens erfolgt durch Gelelektrophorese auf einem 6 %igen Polyacrylamidgel (ohne Harnstoffzusatz, 40 × 20 × 0,1 cm), wobei als Markersubstanz ØX174-DNA (Fa. BRL), geschnitten mit Hinf I, oder pBR 322, geschnitten mit Hae III, dient.

Das so erhaltene Sph I-Eco RI-Fragment wird bei 16° C über Nacht mit dem mit Eco RI und Sph I geschnittenen Vektor pLZPWB2 dMdC ligiert. Nach Transformation kompetenter E. coli-Zellen erhält man Ampicillin-resistente Kolonien, die hinsichtlich Expression eines Fusionsproteins in der erwarteten Größenordnung untersucht werden. Von 4 positiven Klonen wird eine Sequenzanalyse des Angiogenin-Teiles durchgeführt, bei der sich die Sequenz von 2 der 4 Klone als korrekt erweist. Diese Plasmide werden als pLZPWB2 dMdC Ang bezeichnet.

In der gleichen Weise werden die Gene für Angiogenin-Ile-30 bzw. -Val-30 durch Ligation mit den Oligonucleotiden 2c und 2d bzw. 2e und 2f erhalten (Tab. 4).

## Beispiel 6

Die zur gewünschten optischen Dichte kultivierten Bakterienstämme werden mit IPTG als Induktor hinreichend lange, beispielsweise 2 Stunden, induziert. Anschließend werden die Zellen mit 0,1 % Kresol und 0.1 mM Phenylmethyl-sulfonylfluorid (Benzylsulfonylfluorid) abgetötet. Nach Zentrifugieren oder Filtrieren wird die Zellmasse in einer wäßrig-sauren Lösung bei pH 3,0 in einer geeigneten Vorrichtung (French-Presse bzw. ®Dyno-Mühle) aufgeschlossen, worauf die unlöslichen Bestandteile abzentrifugiert werden. Aus dem Überstand werden die Proteine nach Standard-Methoden (Grau et al., Angew. Chem. 98 (1986) 530) isoliert. Durch HPLC-Analytik werden die Anreicherung und die Reinheit der Produkte kontrolliert.

Zur Freisetzung des Angiogenins aus dem Fusionsprotein wird der getrocknete Rückstand in 70 %iger Ameisensäure gelöst umd mit 20 Gewichtsprozent Bromcyan (bezogen auf die eingesetzte Trockenmasse) für 4 h bei RT inkubiert. Durch Fällung mit 5 Volumina Methyl-t-butyl-ether erhält man ein Bromcyan-Spaltprodukt, aus dem durch Chromatographie an geeigneten Kationenaustauschern (z.B. S-Sepharose® bei pH 8 mit 6-8 M Harnstoff) unter reduzierenden Bedingungen (z.B. unter Zusatz von Mercaptoethanol, Dithiothreitol etc.) hochangereicherte Chargen reduzierten Angiogenins isoliert werden können. Für die Faltung des Angiogenins zur nativen Tertiärstruktur unter Schließung der drei Disulfidbrücken finden klassische Methoden der Regeneration reduzierter Proteine mittels geeigneter Mischungen von oxidiertem und reduziertem Glutathion Anwendung (z.B. Karim Ahmed et al., J. Biol. Chem. 250 (1975) 8477-8482). Aus dem Faltungsprodukt wird durch Gelfiltration, gefolgt von Ionenaustauschchromatographie, hochreines Angiogenin isoliert.

Die Reinheitsüberprüfung des Endprodukts erfolgt durch "reversed Phase"-HPLC und SDS-Gelelektrophorese. Die Angiogeneseaktivität wird im CAM-Assay und im Rabbit cornea-Assay (Fett et al., Biochemistry 24 (1985) 5480-5486) nachgewiesen.

0 292 763

**Tabelle 1**

---

<pre>
  SphI                           XhoI    AccI              10                                    Sau96I              PvuI
                                                                                                20
         HIS MET GLN ASP ASN SER ARG TYR THR HIS PHE LEU THR GLN HIS TYR ASP ALA LYS PRO GLN GLY ARG ASP ASP ARG TYR CYS
      C CAC ATG CAG GAC AAC TCG AGG TAT ACA CAT TTC CTG ACC CAG CAC TAT GAC GCT AAA CCG CAG GGC CGG GAC GAT CGT TAC TGC
     GT ACG GTG TAC GTC CTG TTG AGC TCC ATA TGT GTA AAG GAC TGG GTC GTG ATA CTG CGA TTT GGC GTC CCG GCC CTG GTA GCA ATG ACG


         DdeI
         30                              SpeI           40      FokI                   NcoI           50              NdeI
  GLU SER ILE LEU ARG ARG ARG GLY LEU THR SER PRO CYS LYS ASP ILE ASN THR PHE ILE HIS GLY ASN LYS ARG SER ILE LYS ALA ILE
  GAA TCG ATT CTG AGA CGC CGT GGG TTA ACT AGT CCG TGC AAA GAT ATC AAC ACT TTC ATC CAT GGT AAC AAG CGT TCT ATC AAA GCC ATA
  CTT AGC TAA GAC TCT GCG GCA CCC AAT TGA TCA GGC ACG TTT CTA TAG TTG TGA AAG TAG GTA CCA TTG TTC GCA AGA TAG TTT CGG TAT


                60       BstEII                         HhaI
                                                        70                   HindIII          80              BamHI
  CYS GLU ASN LYS ASN GLY ASN PRO HIS ARG GLU ASN LEU ARG ILE SER LYS SER SER PHE GLN VAL THR THR CYS LYS LEU HIS GLY GLY
  TGC GAA AAC AAA AAC GGT AAC CCG CAT CGC GAA AAC CTG CGC ATC AGC AAG TCA AGC TTC CAG GTT ACA ACT TGC AAA CTT CAT GGG GGA
  ACG CTT TTG TTT TTG CCA TTG GGC GTA GCG CTT TTG GAC GCG TAG TCG TTC AGT TCG AAG GTC CAA TGT TGA ACG TTT GAA GTA CCC CCT


         Fnu4HI
         90                       RsaI            NaeI  100                                    110              XbaI   MboII
  SER PRO TRP PRO PRO CYS GLN TYR ARG ALA THR ALA GLY PHE ARG ASN VAL VAL VAL ALA CYS GLU ASN GLY LEU PRO VAL HIS LEU ASP
  TCC CCG TGG CCG CCA TGC CAG TAC CGT GCT ACT GCC GGC TTC CGT AAT GTT GTG GTT GCT TGT GAA AAC GGT CTG CCA GTC CAT CTA GAT
  AGG GGC ACC GGC GGT ACG GTC ATG GCA CGA TGA CGG CCG AAG GCA TTA CAA CAC CAA CGA ACA CTT TTG CCA GAC GGT CAG GTA GAT CTA


                120     StuI                 EcoRI
  GLN SER ILE PHE ARG ARG PRO --- ---
  CAG TCT ATC TTC CGA AGG CCT TAA TAG
  GTC AGA TAG AAG GCT TCC GGA ATT ATC TTA A
</pre>

## Tabelle 2

Mutagene "Primer" für die Umwandlung von Codons für Methionin in.Codons für Leucin bzw. Isoleucin und für die Umwandlung von Codons für Cystein in Codons für Serin:

```
dM1 5'-   AG ACC GTT CAG ACA GAA CTG G
dM2 5'-   AG CGC CAC CAG CCA GTG CAG G
dM3 5'- GCA AAA ATC CAG TTC GCT GGT G
dM4 5'-    C GCC AAT CCA TAT CTG TGA AA
dM5 5'-    C GGT AAT CGC AAT TTG ACC AC
dM6 5'-    A CGG GGT ATA GAT GTC TGA CA
dM7 5'-    G GCT GGT TTC AAT CAG TTG CT
dM8 5'-    C ACC AAT CCC TAT ATG GAA ACC
dC1 5'-    G ACC GTT CAG AGA GAA CTG GCG
dC2 5'-      CAG CTC GAT GGA AAA ATC CAG TTC
dC3 5'-      ATC TGC CGT GGA CTG CAA CAA
dC4 5'-      CGC CAG CTG GGA GTT CAG GCC
dC5 5'-      GCG CTC AAA AGA GGC GGC AGT
dC6 5'-      GCG CGT CCC GGA GCG CAG ACC
```

Tabelle 3

SphI ──────────────────────── 1a ──────────────────────►┤├──────────── 2a ─────────────────

  HIS MET GLN ASP ASN SER ARG TYR THR HIS PHE LEU THR GLN HIS TYR ASP ALA LYS PRO GLN GLY ARG ASP ASP ARG TYR CYS
  ;C CAC ATG CAG GAC AAC TCG AGG TAT ACA CAT TTC CTG ACC CAG CAC TAT GAC GCT AAA CCG CAG GGC CGG GAC GAT CGT TAC TGC
GT ACG GTG TAC GTC CTG TTG AGC TCC ATA TGT GTA AAG GAC TGG GTC GTG ATA CTG CGA TTT GGC GTC CCG GCC CTG CTA GCA ATG ACG

◄──────────────────────── 1b ──────────────────────┤├──────────── 2b ─────────────────


├──────────────────────────── 3a ────────────────────────────►►

GLU SER ILE LEU ARG ARG ARG GLY LEU THR SER PRO CYS LYS ASP ILE ASN THR PHE ILE HIS GLY ASN LYS ARG SER ILE LYS ALA ILE
GAA TCG ATT CTG AGA CGC CGT GGG TTA ACT AGT CCG TGC AAA GAT ATC AAC ACT TTC ATC CAT GGT AAC AAG CGT TCT ATC AAA GCC ATA
CTT AGC TAA GAC TCT GCG GCA CCC AAT TGA TCA GGC ACG TTT CTA TAG TTG TGA AAG TAG GTA CCA TTG TTC GCA AGA TAG TTT CGG TAT

────────────────────────────── 3b ────────────────────────────┤


◄──────────────────────── 4a ────────────────────────►►

CYS GLU ASN LYS ASN GLY ASN PRO HIS ARG GLU ASN LEU ARG ILE SER LYS SER SER PHE GLN VAL THR THR CYS LYS LEU HIS GLY GLY
TGC GAA AAC AAA AAC GGT AAC CCG CAT CGC GAA AAC CTG CGC ATC AGC AAG TCA AGC TTC CAG GTT ACA ACT TGC AAA CTT CAT GGG GGA
ACG CTT TTG TTT TTG CCA TTG GGC GTA GCG CTT TTG GAC GCG TAG TCG TTC AGT TCG AAG GTC CAA TGT TGA ACG TTT GAA GTA CCC CCT

◄──────────────────────── 4b ────────────────────────►├──────── 5b ────────


├──────────── 5a ────────────►◄ ──────────────── 6a ─────────────────

SER PRO TRP PRO PRO CYS GLN TYR ARG ALA THR ALA GLY PHE ARG ASN VAL VAL VAL ALA CYS GLU ASN GLY LEU PRO VAL HIS LEU ASP
TCG CCG TGG CCG CCA TGC CAG TAC CGT GCT ACT GCC GGC TTC CGT AAT GTT GTG GTT GCT TGT GAA AAC GGT CTG CCA GTC CAT CTA GAT
AGG GGC ACC GGC GGT ACG GTC ATG GCA CGA TGA CGG CCG AAG GCA TTA CAA CAC CAA CGA ACA CTT TTG CCA GAC GGT CAG GTA GAT CTA

◄──────────── 5b ────────────►◄ ──────────────── 6b ─────────────────


──────────────────────────────► EcoRI

GLN SER ILE PHE ARG ARG PRO --- ---
CAG TCT ATC TTC CGA AGG CCT TAA TAG
GTC AGA TAG AAG GCT TCC GGA ATT ATC TTA A·

──────────────────────────────►

0 292 763

0 292 763

Tabelle 4

----------------------------------------------------------------

|← ———————————————————————— 2a ————————————————————————→|

```
    TYR ASP ALA LYS PRO GLN GLY ARG ASP ASP ARG TYR CYS GLU SER ILE     ARG ARG ARG GLY
  C TAT GAC GCT AAA CCG CAG GGC CGG GAC GAT CGT TAC TGC GAA TCG ATT NNN AGA CGC CGT GGG T
      T GGC GTC CCG GCC CTG CTA GCA ATG ACG CTT AGC TAA NNN TCT GCG GCA CCC AAT TGA TCA GGC A
```

|← ———————————————— 2b ————————————————→|

|        | Leu |
|--------|-----|
| 2a     | CTG |
| 2b     | GAC |
|        | Ile |
| 2c     | ATC |
| 2d     | TAG |
|        | Val |
| 2e     | GTT |
| 2f     | CAA |

# 0 292 763

## Ansprüche

1. Verfahren zur Herstellung eines Angiogenins, dadurch gekennzeichnet, daß man das Strukturgen für dieses Polypeptid im korrekten Leserahmen über ein Gen für $\beta$-Galactosidase oder ein $\beta$-Galactosidase-Fragment von vorzugsweise über 250 Aminosäuren, jedoch signifikant weniger als die gesamte $\beta$-Galactosidase-Sequenz, vorzugsweise mit etwa 300 bis etwa 800 Aminosäuren, insbesondere mit etwa 320 bis 650 Aminosäuren, wobei im Gen für die $\beta$-Galactosidase oder für das $\beta$-Galactosidase-Fragment Codons für Methionin und/oder Arginin und/oder Cystein ganz oder teilweise durch Codons anderer Aminosäuren ersetzt sind, an eine Regulationsregion koppelt, diese Genstruktur in ein Bakterium einbringt, darin ein unlösliches Fusionsprotein exprimiert, dieses nach einem Zellaufschluß isoliert und das gewünschte Polypeptid durch chemische oder enzymatische Spaltung gewinnt.

2. Verfahren nach Anspruch 1, dadurch gekennzeichnet, daß das $\beta$-Galactosidase-Fragment aus einer Fusion einer aminoterminalen und carboxyterminalen Teilsequenz besteht.

3. Verfahren nach einem oder mehreren der vorhergehenden Ansprüche, dadurch gekennzeichnet, daß das Gen für das $\beta$-Galactosidase-Fragment einer DNA-Sequenz gemäß Figur 1 entspricht.

4. Verfahren nach einem oder mehreren der vorhergehenden Ansprüche, dadurch gekennzeichnet, daß das Strukturgen für das Angiogenin über einen Adaptor an das Gen für das $\beta$-Galactosidase-Fragment gekoppelt ist.

5. Verfahren nach einem oder mehreren der vorhergehenden Ansprüche, dadurch gekennzeichnet, daß unmittelbar vor dem aminoterminalen Ende des Strukturgens ein oder mehrere Codon(s) für eine oder mehrere Aminosäure(n) steht, die eine chemische oder enzymatische Abtrennung des Polypeptids vom $\beta$-Galactosidase-Anteil erlaubt.

6. Verfahren nach einem oder mehreren der vorhergehenden Ansprüche, dadurch gekennzeichnet, daß das Angiogenin durch die DNA-Sequenz nach Tab. 1 oder 4 codiert ist.

7. Strukturgen für ein Angiogenin mit der Aminosäuresequenz 1-123 gemäß Tabelle 1 und 4.

8. DNA-Sequenzen für ein Angiogenin gemäß Tabelle 1 und 4.

9. Genstruktur, enthaltend eine Regulationsregion, ein Gen für $\beta$-Galactosidase oder ein $\beta$-Galactosidase-Fragment gemäß einem oder mehreren der vorhergehenden Ansprüche und ein Strukturgen für ein Angiogenin, wobei das Strukturgen gegebenenfalls über einen Adaptor an das Gen für $\beta$-Galactosidase oder das $\beta$-Galactosidase-Fragment gekoppelt ist, der den korrekten Leserahmen gewährleistet.

10. Vektor, enthaltend eine Genstruktur nach Anspruch 9.

11. Bakterium, vorzugsweise E. coli, enthaltend einen Vektor nach Anspruch 10.

12. Fusionsprotein, enthaltend $\beta$-Galactosidase oder ein $\beta$-Galactosidase-Fragment nach einem oder mehreren der Ansprüche 1 bis 6 und ein Angiogenin.

13. Angiogenin-Derivat mit der Aminosäuresequenz 1 - 123 gemäß Tabelle 1 und 4.

Patentansprüche für den folgenden Vertragsstaat: ES:

1. Verfahren zur Herstellung eines Angiogenins, dadurch gekennzeichnet, daß man das Strukturgen für dieses Polypeptid im korrekten Leserahmen über ein Gen für $\beta$-Galactosidase oder ein $\beta$-Galactosidase-Fragment von vorzugsweise über 250 Aminosäuren, jedoch signifikant weniger als die gesamte $\beta$-Galactosidase-Sequenz, vorzugsweise mit etwa 300 bis etwa 800 Aminosäuren, insbesondere mit etwa 320 bis 650 Aminosäuren, wobei im Gen für die $\beta$-Galactosidase oder für das $\beta$-Galactosidase-Fragment Codons für Methionin und/oder Arginin und/oder Cystein ganz oder teilweise durch Codons anderer Aminosäuren ersetzt sind, an eine Regulationsregion koppelt, diese Genstruktur in ein Bakterium einbringt, darin ein unlösliches Fusionsprotein exprimiert, dieses nach einem Zellaufschluß isoliert und das gewünschte Polypeptid durch chemische oder enzymatische Spaltung gewinnt.

2. Verfahren nach Anspruch 1, dadurch gekennzeichnet, daß das $\beta$-Galactosidase-Fragment aus einer Fusion einer aminoterminalen und carboxyterminalen Teilsequenz besteht.

3. Verfahren nach einem oder mehreren der vorhergehenden Ansprüche, dadurch gekennzeichnet, daß das Gen für das $\beta$-Galactosidase-Fragment einer DNA-Sequenz gemäß Figur 1 entspricht.

4. Verfahren nach einem oder mehreren der vorhergehenden Ansprüche, dadurch gekennzeichnet, daß das Strukturgen für das Angiogenin über einen Adaptor an das Gen für das $\beta$-Galactosidase-Fragment gekoppelt ist.

13

5. Verfahren nach einem oder mehreren der vorhergehenden Ansprüche, dadurch gekennzeichnet, daß unmittelbar vor dem aminoterminalen Ende des Strukturgens ein oder mehrere Codon(s) für eine oder mehrere Aminosäure(n) steht, die eine chemische oder enzymatische Abtrennung des Polypeptids vom β-Galactosidase-Anteil erlaubt.

6. Verfahren nach einem oder mehreren der vorhergehenden Ansprüche, dadurch gekennzeichnet, daß das Angiogenin durch die DNA-Sequenz nach Tab. 1 oder 4 codiert ist.

7. Strukturgen für ein Angiogenin mit der Aminosäuresequenz 1-123 gemäß Tabelle 1 und 4.

8. DNA-Sequenzen für ein Angiogenin gemäß Tabelle 1 und 4.

Patentansprüche für den folgenden Vertragsstaat: GR:

1. Verfahren zur Herstellung eines Angiogenins, dadurch gekennzeichnet, daß man das Strukturgen für dieses Polypeptid im korrekten Leserahmen über ein Gen für β-Galactosidase oder ein β-Galactosidase-Fragment von vorzugsweise über 250 Aminosäuren, jedoch signifikant weniger als die gesamte β-Galactosidase-Sequenz, vorzugsweise mit etwa 300 bis etwa 800 Aminosäuren, insbesondere mit etwa 320 bis 650 Aminosäuren, wobei im Gen für die β-Galactosidase oder für das β-Galactosidase-Fragment Codons für Methionin und/oder Arginin und/oder Cystein ganz oder teilweise durch Codons anderer Aminosäuren ersetzt sind, an eine Regulationsregion koppelt, diese Genstruktur in ein Bakterium einbringt, darin ein unlösliches Fusionsprotein exprimiert, dieses nach einem Zellaufschluß isoliert und das gewünschte Polypeptid durch chemische oder enzymatische Spaltung gewinnt.

2. Verfahren nach Anspruch 1, dadurch gekennzeichnet, daß das β-Galactosidase-Fragment aus einer Fusion einer aminoterminalen und carboxyterminalen Teilsequenz besteht.

3. Verfahren nach einem oder mehreren der vorhergehenden Ansprüche, dadurch gekennzeichnet, daß das Gen für das β-Galactosidase-Fragment einer DNA-Sequenz gemäß Figur 1 entspricht.

4. Verfahren nach einem oder mehreren der vorhergehenden Ansprüche, dadurch gekennzeichnet, daß das Strukturgen für das Angiogenin über einen Adaptor an das Gen für das β-Galactosidase-Fragment gekoppelt ist.

5. Verfahren nach einem oder mehreren der vorhergehenden Ansprüche, dadurch gekennzeichnet, daß unmittelbar vor dem aminoterminalen Ende des Strukturgens ein oder mehrere Codon(s) für eine oder mehrere Aminosäure(n) steht, die eine chemische oder enzymatische Abtrennung des Polypeptids vom β-Galactosidase-Anteil erlaubt.

6. Verfahren nach einem oder mehreren der vorhergehenden Ansprüche, dadurch gekennzeichnet, daß das Angiogenin durch die DNA-Sequenz nach Tab. 1 oder 4 codiert ist.

7. Strukturgen für ein Angiogenin mit der Aminosäuresequenz 1-123 gemäß Tabelle 1 und 4.

8. DNA-Sequenzen für ein Angiogenin gemäß Tabelle 1 und 4.

9. Genstruktur, enthaltend eine Regulationsregion, ein Gen für β-Galactosidase oder ein β-Galactosidase-Fragment gemäß einem oder mehreren der vorhergehenden Ansprüche und ein Strukturgen für ein Angiogenin, wobei das Strukturgen gegebenenfalls über einen Adaptor an das Gen für β-Galactosidase oder das β-Galactosidase-Fragment gekoppelt ist, der den korrekten Leserahmen gewährleistet.

10. Vektor, enthaltend eine Genstruktur nach Anspruch 9.

11. Bakterium, vorzugsweise E. coli, enthaltend einen Vektor nach Anspruch 10.

12. Fusionsprotein, enthaltend β-Galactosidase oder ein β-Galactosidase-Fragment nach einem oder mehreren der Ansprüche 1 bis 6 und ein Angiogenin.

FIG.1

FIG.2

# FIG.3

FIG.4

FIG.5

FIG.6

0 292 763